Europäisches Patentamt

European Patent Office

Office européen des brevets

(11) Veröffentlichungsnummer: **0 312 108 B1**

(12) **EUROPÄISCHE PATENTSCHRIFT**

(19)

(45) Veröffentlichungstag der Patentschrift: **01.07.92**

(21) Anmeldenummer: **88117158.1**

(22) Anmeldetag: **14.10.88**

(51) Int. Cl.⁵: **A61K 49/00**

(54) **Mittel zur Kontrastierung von bösartigen Neubildungen bei ihrer Diagnostik.**

(30) Priorität: **15.10.87 SU 4313068**

(43) Veröffentlichungstag der Anmeldung:
**19.04.89 Patentblatt 89/16**

(45) Bekanntmachung des Hinweises auf die
Patenterteilung:
**01.07.92 Patentblatt 92/27**

(84) Benannte Vertragsstaaten:
**CH DE FR GB IT LI SE**

(56) Entgegenhaltungen:
**DE-A- 1 813 150**

**CHEMICAL ABSTRACTS, Band 107, Nr. 13, 28.
September 1987, Seite 311, Nr. 112022t, Columbus, Ohio, US; O.V. KALAIDZIDIS et al.:
"Selective accumulation of the sodium fluorescein in the malignant cells", & DOKL.
AKAD. NAUK SSSR 1987, 294(3), 715-18**

**PATENT ABSTRACTS OF JAPAN, Band 11,
Nr. 273 (C-445)[2720], 4. September 1987; &
JP-A-62 77 336 (NIPPON SEKIGAISEN KOGYO
K.K.) 09-04-1987**

(73) Patentinhaber: **Bio-Photonics, Inc.
10641 First Street East
Treasure Island Florida 33706(US)**

(72) Erfinder: **Dzbanovsky, Nikolai Nikolaevich
ulitsa V. Ulbrikhta, 8, kv. 95
Moscow(SU)**
Erfinder: **Polsachev, Viktor Iosifovich
Izmailovsky prospekt, 123/I, kv. 29
Moscow(SU)**
Erfinder: **Potemkina, Elena Vasilievna
Frunzenskaya naberezhnaya, 8, kv. 26
Moscow(SU)**
Erfinder: **Rakhimov, Alexandr Tursunovich
Rostovskaya naberezhnaya, 1, kv. 95
Moscow(SU)**
Erfinder: **Rubin, Leonid Borisovich
Lomonosovsky prospekt, 14, kv. 499
Moscow(SU)**
Erfinder: **Osipov, Alexandr Sergeevich
Mosfilmovskaya ulitsa, 32, kv. 42
Moscow(SU)**

(74) Vertreter: **von Füner, Alexander, Dr. et al
Patentanwälte v. Füner, Ebbinghaus, Finck
Mariahilfplatz 2 & 3
W-8000 München 90(DE)**

CHEMICAL ABSTRACTS, Band 106, Nr. 25, 22. Juni 1987, Seite 27, Nr. 207293a, Columbus, Ohio, US; L. CERCEK et al.: "Effects of ascorbate ions on intracellular fluorescein emission polarization spectra in cancer and normal proliferating cells", & CANCER DETECT. PREV. 1987, 10(1-2), 1-20

SOVIET INVENTIONS ILLUSTRATED, Sektion P/O, Woche E27, 8. August 1982, Derwent Publications Ltd, London, GB; & SU-A-792 878 (LENGD NUCLEAR PHYS) 07-02-1982

BRITISH JOURNAL OF OPHTHALMOLOGY, Band 63, Nr. 3, 1979, Seiten 157-160; A.F. BROVKINA et al.: "Value of fluorescein iridography in diagnosis of tumours of the iridociliary zone"

## Beschreibung

Die vorliegende Erfindung bezieht sich auf die Medizin, und zwar auf die Onkologie.

Am besten und sinnvollsten kann die vorliegende Erfindung für die Diagnostik, darunter auch für eine frühe Diagnostik von bösartigen Neubildungen, für die Bestimmung der Verbreitung von bösartigen Schädigungen, die Bestimmung des Umfangs von Operationen, die Präzisierung von Diagnosen in Kliniken und Gesundheitsfürsorgestellen, die Überwachung der Behandlung von bösartigen Geschwulsten und die Feststellung von Nachoperationsrückfällen angewendet werden.

Gegenwärtig stellt das Problem, bösartige Neubildungen, insbesondere im Frühstadium, zu diagnostizieren, eines der aktuellsten Probleme in der Onkologie dar. Trotz einer weiten Anwendung von histologischen Methoden der Analyse, den großen Erfolgen der Ultraschall- und Röntgendiagnostik, der Endoskopieuntersuchungen sowie der Röntgen- und der NMR-Tomografie sind die Empfindlichkeit, die Genauigkeit und Zugänglichkeit dieser Methoden nicht ausreichend. Eine der Richtungen für die Vervollkommnung der Diagnostik von bösartigen Geschwulsten besteht in der Anwendung von darin selektiv akkumulierten konstrastierenden Stoffen. Es ist z.B. gut bekannt, daß bösartige Geschwulste einige Farbstoffe in einer erhöhten Konzentration im Vergleich zu den normalen Geweben akkumulieren, was für eine Fluoreszenzdiagnostik von Geschwulsten nach einer charakteristischen Fluoreszenz des darin akkumulierten Farbstoffes angewendet wird.

Für eine Fluoreszenzkontrastierung hat man verschiedene Verbindungen, z.B. Antibiotica der Gruppe Tetracyclin, angewendet. Eine Methode gestattete es, den Magenkrebs im Spätstadium (II. und IV. Stadium) festzustellen, jedoch mit einer Genauigkeit, die für diagnostische Zwecke nicht ausreichend war (s. Klinger I., Katz K., Gastroenterology, 1961, 41, 29-32). Die Anwendung der Endoskopietechnik in Kombination mit der Tetracyclin-Fluoreszenzkontrastierung (s. Barskij I. Ja., Papajan G.W., Tschedrunow W.W., Gluchin JU.A., Sammelband "Lumineszenzanalyse in medizinisch-biologischen Erforschungen", Riga, 1983, Seiten 182-189) zeigte ebenfalls eine nicht ausreichend hohe Zuverlässigkeit der Diagnostik von Geschwulsten durch einen niedrigen Akkumulierungskontrast von Tetracyclin in bösartigen Geweben im Vergleich zu den normalen Geweben.

Die Fluoreszenzkontrastierung durch den Farbstoff Fluoreszein wurde für die Feststellung von Metastasen in regionären Lymphknoten beim Kehlkopfkrebs angewendet (s. S.I. Mostowoj, Zeitschrift von Ohren-, Nasen- und Halskrankheiten, 1961, Nr. 4, Seiten 34-36). Es wurde hervorgehoben, daß eine helle Fluoreszenz nicht nur durch den Krebsvorgang geschädigte Lymphknoten, sondern auch andere Organe aufwiesen, was diese Methode für die Diagnostik unbrauchbar machte. Zu Analogieschlüssen sind auch die Autoren einer Reihe von anderen Arbeiten gekommen (s. Moore G.E. Science 1947, 106, 130-131, Figge F.H.I., Weinland G.S., Maganiello L.O.I., Proc. Soc. Exper. Biol. Med. 68, 640-641, 1948).

Man hat auch versucht, ein Mittel zur Kontrastierung von bösartigen Geschwulsten zu entwickeln, welches als Kontrastierungsstoff das Fluoreszein enthielt. Dieses wurde entweder als 20%ige Lösung (2 bis 5 ml für eine intravenöse Einführung) oder in Form eines Pulvers (1 g), auf welches Sodawasser getrunken wurde, angewendet ( s. Ju.N. Efuni, Zeitschrift "Vestnik otorinolaringologii", 1961, Nr. 2, Seiten 11-15). Ein Nachteil dieses Kontrastmittels ist ein niedriger Kontrast der Akkumulierung von Fluoreszein in einem bösartigen Gewebe im Vergleich zu einem normalen Gewebe, was die Genauigkeit der Diagnostik verringert. Der Akkumulierungskontrast des Fluoreszeins in der bösartigen Geschwulst im Vergleich zum normalen Gewebe wurde durch ein Verhältnis der Konzentration des darin enthaltenen Fluoreszeins pro Gramm der Masse des Gewebes bestimmt. Nach den Literaturangaben betrug die Richtigkeit der Fluoreszenzdiagnostik von bösartigen Geschwulsten, z.B. von Ohren-, Nasen- und Halsgeschwulsten, 30 %. Man kam zu dem Ergebnis, daß epitheliale bösartige Geschwulste nicht fluoreszieren, d.h. daß sie das Fluoreszein nicht akkumulieren.

Die Aufgabe der vorliegenden Erfindung besteht darin, ein Mittel zur Kontrastierung von bösartigen Neubildungen bereitzustellen, welches eine hohe Zuverlässigkeit der Diagnostik gewährleisten würde. Die gestellte Aufgabe wurde unerwartet durch die Anwendung im Mittel zur Kontrastierung neben dem Fluoreszein oder seinen Salzen oder seinem Anion von Zusatzstoffen in Form von Sacchariden und/oder Disacchariden, Vitaminen und Antihistaminica gelöst. Der Wirkungsmechanismus dieser Zusatzstoffe zusammen mit dem Fluoreszein, der im voraus absolut nicht offensichtlich war, besteht, soweit feststellbar, in folgendem.

Das Fluoreszein wird als ein fluoreszierender Farbstoff in der Biologie und der Medizin schon seit langem angewendet. Es ist allgemein bekannt, daß das Fluoreszein in lebende Zellen nicht durchdringt, weil sein Anion über eine negative Ladung verfügt, welche das Durchdringen durch die Zellmembrane verhindert. Es wurde unerwartet festgestellt, daß, wenn man der Fluoreszenzlösung organische Säuren, welche Produkt des Zellmetabolismus sind, zugibt, dies dazu führt, daß das Fluoreszein beginnt, in die Zellen einzudringen und sich in diesen durch die Bildung eines Komplexes, welcher die Ladung des Fluoreszeinanions neutralisiert, zu akkumulieren. Dieser Effekt wurde in der vorliegenden Erfindung für eine Fluoreszenzdiagnostik benutzt. Bösartige Zellen verfügen über einen glykolytischen Typ der Atmung, für welchen ein erhöhter Verbrauch von Kohlenhydraten und eine Entsäuerung der Zwischengewebeflüssigkeit durch den Auswurf von organischen Säuren als Endprodukt der Atmung charakteristisch sind. Wie die Forschungen gezeigt haben, führt die Senkung des Säuregehaltes des Mediums zur Protenisation des Fluoreszeins, zur Erhöhung der Lösbarkeit in Zellmembranen und folglich zu einer starken Erhöhung seiner Konzentration innerhalb bösartiger Zellen.

Gegenstand der Erfindung ist ein Mittel zur Kontrastierung von bösartigen Neubildungen bei ihrer Diagnostik, enthaltend Fluoreszein und/oder seine Salze als Kontraststoff, das dadurch gekennzeichnet ist, daß dieses zusätzlich Saccharide und/oder Disaccharide in folgenden Gew.-% enthält:

| Fluoreszein oder seine Salze | 0,1 bis 80 |
|---|---|
| Saccharide und/oder Disaccharide | 20 bis 99,9. |

Bevorzugt ist ein Mittel folgender Zusammensetzung in Gew.-%:

| Fluoreszein oder seine Salze | 10 |
|---|---|
| Saccharide und/oder Disaccharide | 90. |

Gegenstand der Erfindung ist ferner ein Mittel zur Kontrastierung von bösartigen Neubildungen bei ihrer Diagnostik enthaltend Fluoreszein oder seine Salze als Kontraststoff, das dadurch gekennzeichnet ist, daß dieses zusätzlich Vitamine in folgenden Gew.-% enthält:

| Fluoreszein oder seine Salze | 1 bis 90 |
|---|---|
| Vitamine | 10 bis 99. |

Bevorzugt ist ein Mittel, das Vitamine in folgendem Verhältnis in Gew.-% enthält:

| Fluoreszein oder seine Salze | 50 |
|---|---|
| Vitamine | 50. |

Gegenstand der Erfindung ist ferner ein Mittel zur Kontrastierung von bösartigen Neubildungen bei ihrer Diagnostik, enthaltend Fluoreszein oder seine Salze als Kontraststoff, das dadurch gekennzeichnet ist, daß dieses zusätzlich Antihistaminica in folgenden Gew.-% enthält:

| Fluoreszein oder seine Salze | 20 bis 90 |
|---|---|
| Antihistaminica | 10 bis 80. |

Bevorzugt ist ein Mittel, das Antihistaminica in folgendem Verhältnis in Gew.-% enthält:

| Fluoreszein oder seine Salze | 80 |
|---|---|
| Antihistaminica | 20. |

Gegenstand der Erfindung ist ferner ein Mittel zur Kontrastierung von bösartigen Neubildungen bei ihrer Diagnostik, enthaltend Fluoreszein oder seine Salze als Kontraststoff, das dadurch gekennzeichnet ist, daß dieses zusätzlich Saccharide und/oder Disaccharide, Vitamine und Antihistaminica in folgenden Gew.-% enthält:

| Fluoreszein oder seine Salze | 0,1 bis 79,6 |
|---|---|
| Saccharide und/oder Disaccharide | 19,4 bis 98,9 |
| Vitamine | 0,5 bis 80 |
| Antihistaminica | 0,5 bis 80. |

Bevorzugtist ein Mittel, das Saccharide und/oder Disaccharide, Vitamine und Antihistaminica in folgendem Verhältnis in Gew.-% enthält:

| Fluoreszein oder seine Salze | 15 |
|---|---|
| Saccharide und/oder Disaccharide | 70 |
| Vitamine | 10 |
| Antihistaminica | 5. |

Bevorzugt ist ferner ein Mittel, das als Fluoreszeinsalze Alkalimetallsalze enthält.

Bevorzugt ist ferner ein Mittel, das als Fluoreszeinsalze seine Ammoniumsalze enthält.

Bevorzugt ist ferner ein Mittel, das als Alkalimetallsalze des Fluoreszeins Li-, Na- und K-Salze enthält.

Bevorzugt ist ferner ein Mittel, das als Saccharide Glukose und/oder Fruktose enthält.

Bevorzugt ist ferner ein Mittel, das als Disaccharide Saccharose enthält.

Bevorzugt ist ferner ein Mittel, das als Vitamine die Vitamine der Gruppe A und/oder die Vitamine der Gruppe B und/ oder die Vitamine der Gruppe C und/oder P und/oder PP und/ oder E enthält.

Bevorzugt ist ferner ein Mittel, das als Vitamine die Vitamine der Gruppe B und/oder die Vitamine der Gruppe C und/ oder P und/oder PP und oder E enthält.

Bevorzugt ist ferner ein Mittel, das als Antihistaminica 3-Methyl-9-benzyl-1,2,4-tetrahydrocarbolin-naphthalin-1,5-disulfonat,Hydrochlorid des 10-(2-Dimethylaminopropyl)-phenothiazins, Hydrochlorid des N-Dimethylaminoethyl-N-(para-chlorbenzyl)-aminopyridins oder 1-Methyl-2[2-($\alpha$-methyl-para-chlorbenzhydry-loxy)-ethyl]-pyrrolidins, 4,9-Dihydro-4(1-methyl-4-piperidinyliden)-1-OH-benzo[4,5]cyclohepta[1,2]thiophen-10-OH(Hydrofumarat), Hydrochlorid des $\beta$-Dimethylaminoethylethers des Benzhydrols oder des Chinuklydyl-3-diphenyl-carbinols enthält.

Bevorzugt ist ferner ein Mittel, das dadurch gekennzeichnet ist, daß es als Lösung oder Pulver vorliegt.

Bevorzugt ist ferner ein Mittel, das dadurch gekennzeichnet ist, daß es als Lösungsmittel Wasser oder wässerige 5 bis 30%ige Lösung des Ethylalkohols enthält.

Als Kohlehydrate wählt man vorzugsweise Glukose oder Fruktose als Monosaccharide sowie Saccharose als Disaccharide bei konkreten physiologischen Besonderheiten des Organismus, z.B. wenn der Patient an Diabetes leidet.

Der Zusatz an Vitaminen, und zwar des A-Retinols, des $B_1$-Thiamins, des $B_2$-Riboflavins, des $B_6$-Pyridoxin-Chlorids, des P-Rutins, E-Tokopherol-Acetats, der C-Ascorbinsäure und der PP-Nikotinsäure führt zu einer allgemeinen Stimulierung von metabolytischen Prozessen, die für den aktiven Fluoreszeintransport in bösartige Zellen erforderlich sind, insbesondere der Glykolyse.

Die Anwendung der Antihistaminica beispielsweise von 3-Methyl-9-benzyl-1,2,3,4-tetrahydrocarbolin-naphthalin-1,5-disulfonat oder Hydrochlorid des 10-(2-Dimethylaminopropyl)-phenothiazins, Hydrochlorid des N-Dimethyl-aminoethyl-N-(para-chlorbenzyl)aminopyridins oder 1-Methyl-2/2($\alpha$-methyl-para-chlorbenzh-ydryloxy)-ethyl/-pyrrolidins, 4,9-Dihydro-4-(1-methyl-4-piperidinyliden)1-OH-benzo/4,5/-cyclohepta/1,2/thiophen-10-OH(hydrofumarat), Hydrochlorid des $\beta$-Dimethylaminoethylethers des Benzhydrols oder des Chinuklydyl-3-diphenylcarbinols ist auf die Verringerung der Geschwindigkeit des Austritts des Fluoreszeins aus den bösartigen Zellen gerichtet. Dabei wird auch die Erhöhung der Konzentration des Fluoreszeins in den bösartigen Zellen gewährleistet und als Folge der positive Effekt, und zwar ein erhöhter Kontrast der Akkumulierung des Fluoreszeins in einem bösartigen Gewebe erreicht. Somit gewährleisten die angewandten Zusatzstoffe sowohl die Erhöhung einer selektiven Akkumulierung des Fluoreszeins als auch die Senkung der Geschwindigkeit seines Austritts aus den Zellen. Es sei hervorzuheben, daß die Selektivität dieses Prozesses, die durch die Besonderheiten des Metabolismus von bösartigen Zellen bedingt ist, sich im ganzen in einem hohen Kontrast der Akkumulierung des Fluoreszeins in bösartigen Geweben im Vergleich zu den normalen Geweben gezeigt hat.

Unter Berücksichtigung von individuellen Besonderheiten des Organismus ist es möglich, folgende Varianten des Mittels mit folgender Zusammensetzung (in Gew.-%) anzuwenden:

| 1. Fluoreszein oder seine Salze | 0,1 bis 80 Gew.-% |
|---|---|
| Saccharide und/oder Disaccharide | 20 bis 99,9 " |

Die bevorzugte Zusammensetzung ist die folgende:

| Fluoreszein oder seine Salze | 10 Gew.-% |
|---|---|
| Saccharide und/oder Disaccharide | 90 Gew.-%. |

## Tabelle 1

| Zusammensetzung des Kontrastmittels in Gew.-% | | | | Akkumulierungs- kontrast in rel. Einheiten |
|---|---|---|---|---|
| Fluores- zein oder seine Salze | Glu- kose | Fruk- tose | Saccha- rose | |
| **Bekannte Zusammen- setzung (zum Ver- gleich)** 100 | - | - | - | 1,5 |
| 20 | - | 80 | - | 5,0 |
| 1 | - | - | 99 | 8,5 |
| 0,1 | 99,9 | - | - | 12,0 |
| 3 | 50 | - | 47 | 8,0 |

| 2. Fluoreszein oder seine Salze | 1 bis 90 Gew.-% |
|---|---|
| Vitamine | 10 bis 99 Gew.-%. |

Die bevorzugte Zusammensetzung ist die folgende:

| Fluoreszein oder seine Salze | 50 Gew.-% |
|---|---|
| Vitamine | 50 Gew.-%. |

## Tabelle 2

| Zusammensetzung des Kontrastmittels in Gew.-% | | | | | | | | | | | Akkumu-lierungs-kontrast in rel. Einheiten |
|---|---|---|---|---|---|---|---|---|---|---|---|
| Fluo-res-zein oder seine Salze | Vitamine | | | | | | | | | | |
| | $B_1$ | $B_2$ | $B_6$ | A | P | E | C | PP | | | |
| 1 | 2 | 3 | 4 | 5 | 6 | 7 | 8 | 9 | 10 | | 11 |
| Bekannte Zusammen-setzung (zum Vergl.) | 100 | – | – | – | – | – | – | – | – | | 1,5 |

Tabelle 2 (Fortsetzung)

| 1 | 2 | 3 | 4 | 5 | 6 | 7 | 8 | 9 | 10 | 11 |
|---|---|---|---|---|---|---|---|---|---|---|
| | 90 | 2 | 1 | 2 | 0,2 | 2 | 1 | 1 | 0,8 | 3,0 |
| | 50 | 3 | 1,5 | 3 | 0,5 | 3 | 1,5 | 30 | 7,5 | 6,5 |
| | 10 | 5,4 | 2,7 | 5,4 | 0,9 | 5,4 | 2,7 | 54 | 13,5 | 5,0 |
| | 10 | 5,4 | 2,7 | – | – | 5,4 | 2,7 | 60,3 | 13,5 | 4,5 |

| 3. Fluoreszein oder seine Salze | 20 bis 90 Gew.-% |
|---|---|
| Antihistaminica | 10 bis 80 Gew.-%- |

Die bevorzugte Zusammensetzung ist die folgende:

| Fluoreszein oder seine Salze | 80 Gew.-% |
|---|---|
| Antihistaminica | 20 Gew.-% |

## Tabelle 3

| Zusammensetzung des Kontrastmittels in Gew.-% | | | Akkumulie-rungskon-trast in rel.Einheiten |
|---|---|---|---|
| | Fluoreszein oder seine Salze | 3-Methyl-9-benzyl-1,2,3,4-tetrahydrocarbolin-naph-thalin-1,5-disulfonat | |
| Bekannte Zusammen-setzung (zum Ver-gleich) | 100 | - | 1,5 |
| | 90 | 10 | 4,5 |
| | 50 | 50 | 3,0 |
| | 10 | 90 | 2,5 |

Es sind im Kontrastmittel auch andere Kombinationen der oben aufgezählten Komponenten untereinander möglich. Aus allen möglichen Kombinationen ist folgende volle Zusammensetzung des Mittels (in Gew.-%) bevorzugt:

| Fluoreszein oder seine Salze | 0,1 bis 79,6 |
|---|---|
| Saccharid und/oder Disaccharid | 19,4 bis 98,9 |
| Vitamine | 0,5 bis 80 |
| Blockatoren | 0,5 bis 80. |

Die bevorzugte Zusammensetzung ist folgende:

| Fluoreszein oder seine Salze | 15 Gew.-% |
|---|---|
| Saccharid und/oder Disaccharid | 70 Gew.-% |
| Vitamine | 10 Gew.-% |
| Blockatoren | 5 Gew.-%. |

Tabelle 4

Zusammensetzung des Kontrastmittels , in Gew.-%

| | Fluoreszein oder seine Salze | Glukose | Fruktose | Saccharose | B₁ | B₂ | B₆ | C | A | 3-Methyl-3-benzyl-1,2,3,4-tetrahydrocarbolin-naphthalin-1,5-disulfonat | Akkumulierungskontrast in relativen Einheiten |
|---|---|---|---|---|---|---|---|---|---|---|---|
| | | | | | | Vitamine | | | | | |
| Bekannte Zusammensetzung (zum Vergleich) | 100 | – | – | – | – | – | – | – | – | – | 1,5 |
| | 10 | – | – | – | – | – | – | 60 | – | 30 | 5 |
| | 10 | – | – | – | 5 | 5 | 10 | 20 | – | 50 | 6 |
| | 5 | – | – | 50 | 5 | 5 | 5 | 25 | 5 | – | 10 |
| | 0,1 | 98 | – | – | – | – | – | 1 | 0,4 | 0,5 | 12 |
| | 1 | – | 35 | – | 10 | 10 | 10 | 10 | – | 24 | 18 |
| | 25 | – | 35 | – | 5 | 5 | 5 | 15 | 5 | 5 | 20 |

Wird eine der Komponenten des Kontrastmittels nach dem oben angeführten Schema ausgeschlossen, senkt sich der Kontrast seiner Akkumulierung in bösartigen Geschwulsten etwas, bleibt aber trotzdem auf einem höheren Niveau im Vergleich zum Stand der Technik (s. Tabellen 1 bis 4).

Es ist prinzipiell wichtig, daß als wirksames Agens im Kontrastmittel Fluoreszeinanion ist, welches, indem es sich im Blut des Patienten befindet, durch die Gewebe transportiert wird und sich in bösartigen Neubildungen akkumuliert. Im Zusammenhang damit können im erfindungsgemäßen Kontrastmittel entwe-

9

der Fluoreszein oder seine Salze, vorzugsweise die Salze der Alkalimetalle oder Ammonium, die in einem alkalischen Medium unter Bildung des Fluoreszeinanions dissoziieren, angewendet werden.

Man kann die erfindungsgemäßen Mittel durch intravenöse Injektionen, enteral oder über den Anus einführen. Es gibt auch andere Varianten: man kann z.B. die Vitamine und die Kohlenhydrate enteral einnehmen und das Fluoreszein oder seine Salze intravenös einführen.

Einzelne Komponenten des Kontrastmittels können entweder in Form einer einheitlichen Lösung oder eines Pulvers oder einzeln in Form von individuellen Lösungen oder in fester Form angewendet werden.

Als Lösungsmittel für das Kontrastmittel im ganzen oder für seine einzelnen Komponenten kann Wasser oder eine 5 bis 30%ige wässerige ethanolische Lösung angewendet werden. Man kann auch Salze und Säuren zusetzen, um eine optimale Azidität des Mediums zu schaffen, sowie Geschmacksstoffe.

Man kann das Lösungsmittel entweder zusammen mit dem Mittel (Mittel in Form einer Lösung) oder getrennt (Mittel in Form eines Pulvers plus Lösungsmittel) anwenden.

Die Bestimmung der Fähigkeit des Fluoreszeins oder seiner Salze, sich in bösartigen Zellen und Geschwulsten zu akkumulieren, wurde auf Zellkulturen der He-, La-, STS-, der IG-Fibroplaste, welche nach Standardmethoden kultiviert werden, durchgeführt. Nachdem der Zellkultur das Fluoreszein oder seine Salze neben den anderen Komponenten des Mittels zugesetzt und innerhalb von einigen Stunden inkubiert worden waren, wurde eine Zellfraktion ausgeschieden und nach dem Waschen in den Zellen die Konzentration des Farbstoffes unter Anwendung der Methoden einer chromatografischen und einer Fluoreszenzanalyse bestimmt.

In Versuchen mit Labortieren wurden Mäuse CBA, C-57 Black und BALB-C mit umgeimpften Geschwulsten carciona cervis, Lewis tumor, carciona intestinum crassum getestet. Nach einer bestimmten Zeit nach der Einführung des Fluoreszeins oder seiner Salze neben den anderen Komponenten des Mittels wurden die Tiere unter Narkose getötet und die Konzentration des Farbstoffes in der Geschwulst und in normalen Geweben bestimmt. Der Akkumulierungskontrast wurde als Verhältnis der Konzentrationen im bösartigen Gewebe zum normalen Gewebe bestimmt.

Bei laparoskopischen Untersuchungen wurde festgestellt, daß sich das Fluoreszein oder seine Salze in der Aszitflüssigkeit akkumulieren, was es gestattet, diese in sehr geringen Mengen zu entdecken und zum Schluß zu kommen, daß es in Organen bösartige Tiefmetastasen gibt, die visuell nicht entdeckt werden können, z.B. in der Leber, im Bauchgebiet, in der Milz u.a., was diagnostische Möglichkeiten der Laparoskopiemethode wesentlich erweitert.

In den Tabellen 1 bis 4 sind Ergebnisse der Experimente auf Mäusen der Linie CBA mit dem auf den Schenkel geimpften Gebärmutterkrebs mit der Bestimmung des Akkumulierungskontrastes des Fluoreszeins in der bösartigen Geschwulst im Vergleich zum normalen Umgebungsgewebe in Abhängigkeit von der Zusammensetzung des Kontrastmittels angeführt.

Die im Blut anwesenden bösartigen Elemente akkumulieren das Fluoreszein oder seine Salze und können nach der Fluoreszenzkontrastierung unter dem Mikroskop entdeckt werden.

Man begann mit der Untersuchung der Lokalisation des Fluoreszeins im Gewebe des Organismus nach einer bestimmten Zeit nach der Einführung des Mittels, da innerhalb dieser Zeit, der Farbstoff sich zuerst in allen Geweben gleichmäßig verbreitet und erst danach in den bösartigen Geschwulsten akkumuliert. Der Austritt aus den normalen Geweben über die Leber und die Nieren beginnt praktisch sofort nach der Einführung des Mittels. Nach einer bestimmten Zeit, die für die Lokalisation, den Typ und die Größe der bösartigen Geschwulst charakteristisch ist, wird darin eine erhöhte Konzentration des Fluoreszeins oder seiner Salze festgestellt. Die Feststellung der Lokalisation des Fluoreszeins wurde durch Fluoreszenzanalyse durchgeführt. Die Gewebe mit erhöhten und erniedrigten Konzentrationen des Fluoreszeins wurden einer histologischen Analyse zum Nachweis ihrer bösartigen Natur unterworfen.

Es wurde festgestellt, daß die Richtigkeit der Diagnostik eines bösartigen Vorgangs zwecks Bestimmung der Fluoreszenz in Geweben im Vergleich zu einer üblichen histologischen Analyse eines Biopsiematerials 75 % bis 90 % in Abhängigkeit von der Lokalisation der Geschwulst beträgt.

Analoge Ergebnisse hinsichtlich der Richtigkeit der Fluoreszenzdiagnostik wurden bei klinischen Untersuchungen erzielt, wo insgesamt 350 Kranke, die am Krebs des Verdauungssystems, am Hautkrebs und am Milchdrüsenkrebs litten, untersucht.

**Patentansprüche**

1. Mittel zur Kontrastierung von bösartigen Neubildungen bei ihrer Diagnostik, enthaltend Fluoreszein und/oder seine Salze als Kontraststoff, dadurch **gekennzeichnet,** daß dieses zusätzlich Saccharide und/oder Disaccharide in folgenden Gew.-% enthält:

| Fluoreszein oder seine Salze | 0,1 bis 80 |
| Saccharide und/oder Disaccharide | 20 bis 99,9. |

2. Mittel nach Anspruch 1, dadurch **gekennzeichnet,** daß dieses Saccharide und/oder Disaccharide in folgenden Gew.-% enthält:

| Fluoreszein oder seine Salze | 10 |
| Saccharide und/oder Disaccharide | 90. |

3. Mittel zur Kontrastierung von bösartigen Neubildungen bei ihrer Diagnostik, enthaltend Fluoreszein oder seine Salze als Kontraststoff, dadurch **gekennzeichnet,** daß dieses zusätzlich Vitamine in folgenden Gew.-% enthält:

| Fluoreszein oder seine Salze | 1 bis 90 |
| Vitamine | 10 bis 99. |

4. Mittel nach Anspruch 3, dadurch **gekennzeichnet,** daß dieses Vitamine in folgenden Gew.-% enthält:

| Fluoreszein oder seine Salze | 50 |
| Vitamine | 50. |

5. Mittel zur Kontrastierung von bösartigen Neubildungen bei ihrer Diagnostik, enthaltend Fluoreszein oder seine Salze als Kontraststoff, dadurch **gekennzeichnet,** daß dieses zusätzlich Antihistaminica in folgenden Gew.-% enthält:

| Fluoreszein oder seine Salze | 20 bis 90 |
| Antihistaminica | 10 bis 80. |

6. Mittel nach Anspruch 5, dadurch **gekennzeichnet,** daß dieses Antihistaminica in folgenden Gew.-% enthält:

| Fluoreszein oder seine Salze | 80 |
| Antihistaminica | 20. |

7. Mittel zur Kontrastierung von bösartigen Neubildungen bei ihrer Diagnostik, enthaltend Fluoreszein oder seine Salze als Kontraststoff, dadurch **gekennzeichnet,** daß dieses zusätzlich Saccharide und/oder Disaccharide, Vitamine und Antihistaminica in folgenden Gew.-% enthält:

| Fluoreszein oder seine Salze | 0,1 bis 79,6 |
| Saccharide und/oder Disaccharide | 19,4 bis 98,9 |
| Vitamine | 0,5 bis 80 |
| Antihistaminica | 0,5 bis 80. |

8. Mittel nach Anspruch 7, dadurch **gekennzeichnet,** daß dieses Saccharide und/oder Disaccharide,

EP 0 312 108 B1

Vitamine und Antihistaminica in folgenden Gew.-% enthält:

| Fluoreszein oder seine Salze | 15 |
| Saccharide und/oder Disaccharide | 70 |
| Vitamine | 10 |
| Antihistaminica | 5. |

9.  Mittel nach einem der Ansprüche 1 bis 8, dadurch **gekennzeichnet,** daß als Fluoreszeinsalze Alkalimetallsalze enthalten sind.

10.  Mittel nach einem der Ansprüche 1 bis 8, dadurch **gekennzeichnet,** daß als Fluoreszeinsalze seine Ammoniumsalze enthalten sind.

11.  Mittel nach Anspruch 9, dadurch **gekennzeichnet,** daß als Salze der Alkalimetalle des Fluoreszeins Li-, Na- und K-Salze enthalten sind.

12.  Mittel nach einem der Ansprüche 1, 5 bis 11, dadurch **gekennzeichnet,** daß als Saccharide Glukose und/ oder Fruktose enthalten sind.

13.  Mittel nach einem der Ansprüche 1, 5 bis 11, dadurch **gekennzeichnet,** daß als Disaccharide Saccharose enthalten ist.

14.  Mittel nach einem der Ansprüche 3 und 7 bis 13, dadurch **gekennzeichnet,** daß als Vitamine die Vitamine der Gruppe A und/oder die Vitamine der Gruppe B und/oder die Vitamine der Gruppe C und/oder P und/oder PP und/oder E enthalten sind.

15.  Mittel nach einem der Ansprüche 3 und 7 bis 13, dadurch **gekennzeichnet,** daß als Vitamine die Vitamine der Gruppe B und/oder die Vitamine der Gruppe C und/oder P und/oder PP und/oder E enthalten sind.

16.  Mittel nach einem der Ansprüche 5 und 7 bis 15, dadurch **gekennzeichnet,** daß als Antihistaminica 3-Methyl-9-benzyl-1,2,4-tetrahydrocarbolin-naphthalin-1,5-disulfonat,Hydrochlorid des 10-(2-Dimethylaminopropyl)-phenothiazins, Hydrochlorid des N-Dimethylaminoethyl-N-(para-chlorbenzyl)aminopyridins oder 1-Methyl-2[2-($\alpha$-methylpara-chlorbenzhydryloxy)-ethyl]-pyrrolidins, 4,9-Dihydro-4(1-methyl-4-piperi-dinyliden)-1-OH-benzo[4,5]cyclohepta[1,2] thiophen-10-OH(hydrofumarat), Hydrochlorid des $\beta$-Dimethy-laminoethylethers des Benzhydrols oder des Chinuklydyl-3-diphenyl-carbinols enthalten sind.

17.  Mittel nach einem der Ansprüche 1 bis 16, dadurch **gekennzeichnet,** daß dieses als Lösung oder Pulver vorliegt.

18.  Mittel nach Anspruch 17, dadurch **gekennzeichnet,** daß als Lösungsmittel Wasser oder wässerige 5 bis 30%ige Lösung des Ethylalkohols vorliegt.

## Claims

1.  Contrast medium for the diagnosis of malignant growths, containing fluorescein and/or its salts as contrast substance, characterised in that it additionally contains saccharides and/or disaccharides in the following percentages by weight:

| fluorescein or its salts | 0. 1 to 80 |
| saccharides and/or disaccharides | 20 to 99. 9. |

2.  Medium as claimed in claim 1, characterised in that it contains saccharides and/or disaccharides in the following percentages by weight:

| fluorescein or its salts | 10 |
|---|---|
| saccharides and/or disaccharides | 90. |

3. Contrast medium for the diagnosis of malignant growths, containing fluorescein or its salts as contrast substance, characterised in that it additionally contains vitamins in the following percentages by weight:

| fluorescein or its salts | 1 to 90 |
|---|---|
| vitamins | 10 to 99. |

4. Medium as claimed in claim 3, characterised in that it contains vitamins in the following percentages by weight:

| fluorescein or its salts | 50 |
|---|---|
| vitamins | 50. |

5. Contrast medium for the diagnosis of malignant growths, containing fluorescein or its salts as contrast substance, characterised in that it additionally contains antihistamine in the following percentages by weight:

| fluorescein or its salts | 20 to 90 |
|---|---|
| antihistamine | 10 to 80. |

6. Medium as claimed in claim 5, characterised in that it contains antihistamine in the following percentages by weight:

| fluorescein or its salts | 80 |
|---|---|
| antihistamine | 20. |

7. Contrast medium for the diagnosis of malignant growths, containing fluorescein or its salts as contrast substance, characterised in that it additionally contains saccharides and/or disaccharides, vitamins and antihistamine in the following percentages by weight:

| fluorescein or its salts | 0.1 to 79.6 |
|---|---|
| saccharides and/or disaccharides | 19.4 to 98.9 |
| vitamins | 0.5 to 80 |
| antihistamine | 0.5 to 80. |

8. Medium as claimed in claim 7, characterised in that it contains saccharides and/or disaccharides, vitamins and antihistamine in the following percentages by weight:

| fluorescein or its salts | 15 |
|---|---|
| saccharides and/or disaccharides | 70 |
| vitamins | 10 |
| antihistamine | 5. |

9. Medium as claimed in one of claims 1 to 8, characterised in that alkali metal salts are contained as fluorescein salts.

10. Medium as claimed in one of claims 1 to 8, characterised in that its ammonium salts are contained as fluorescein salts.

11. Medium as claimed in claim 9, characterised in that lithium, sodium and potassium salts are contained as salts of the alkali metal of fluorescein.

12. Medium as claimed in one of claims 1, 5 to 11, characterised in that glucose and/or fructose are contained as saccharides.

13. Medium as claimed in one of claims 1, 5 to 11, characterised in that saccharose is contained as disaccharide.

14. Medium as claimed in one of claims 3 and 7 to 13, characterised in that the vitamins of group A and/or the vitamins or group B and/or the vitamins of group C and/or P and/or PP and/or E are contained as vitamins.

15. Medium as claimed in one of claims 3 and 7 to 13, characterised in that the vitamins of group B and/or the vitamins of group C and/or P and/or PP and/or E are contained as vitamins.

16. Medium as claimed in one of claims 5 and 7 to 15, characterised in that 3-methyl-9-benzyl-1,2,4-tetrahydrocarbolin-naphthalene-1,5-disulphonate, 10-(2-dimethylaminopropyl)-phenothiazine hydrochloride, hydrochloride of N-dimethylaminoethyl-N-para-chlorobensyl)aminopyridine or of 1-methyl-2[2-($\alpha$-methylpara-chlorobenzhydryloxy)-ethyl]-pyrrolidine, 4,9-dihydro-4(1-methyl-4-piperidinylidene)-1-OH-benzo[4,5]cyclohepta(1,2]thiophene-10-OH(hydrofumarate), hydrochloride of $\beta$-dimethylaminoethylether of benzhydrol or of quinuclydyl-3-diphenyl-carbinol are contained as antihistamine.

17. Medium as claimed in one of claims 1 to 16, characterised in that it is present as a solution or powder.

18. Medium as claimed in claim 17, characterised in that water or aqueous 5 to 30% solution of ethyl alcohol is present as solvent.

**Revendications**

1. Moyen de contraste de néoplasmes malins dans leur diagnostic, comprenant de la fluorescéine et/ou son sel en tant qu'agent de contraste, caractérisé en ce que ledit moyen comprend aussi des saccharides et/ou des disaccharides en pourcentage pondéral suivant:

| fluorescéine ou son sel | de 0,1 à 80% |
| saccharide et/ou disaccharide | de 20 à 99,9% |

2. Moyen de contraste selon la revendication 1, caractérisé en ce que ledit moyen comprend des saccharides et/ou des disaccharides en pourcentage pondéral suivant:

| fluorescéine ou son sel | 10% |
| saccharide et/ou disaccharide | 90% |

3. Moyen de contraste de néoplasmes malins dans leur diagnostic comprenant de la fluorescéine et/ou son sel en tant qu'agent de contraste, caractérisé en ce que ledit moyen comprend de plus des vitamines en pourcentage pondéral suivant:

14

| fluorescéine ou son sel | de 1 à 90% |
|---|---|
| vitamines | de 10 à 99% |

**4.** Moyen de contraste selon la revendication 3, caractérisé en ce que ledit moyen comprend des vitamines en pourcentage pondéral suivant:

| fluorescéine ou son sel | 50% |
|---|---|
| vitamines | 50% |

**5.** Moyen de contraste de néoplasmes malins dans leur diagnostic, comprenant de la fluorescéine ou son sel en tant qu'agent de contraste, caractérisé en ce que ledit moyen comprend en outre des anti-histaminiques en pourcentage pondéral suivant:

| fluorescéine ou son sel | de 20 à 90% |
|---|---|
| anti-histaminique | de 10 à 80% |

**6.** Moyen de contraste selon la revendication 5, caractérisé en ce que ledit moyen contient des anti-histaminiques en pourcentage pondéral suivant:

| fluorescéine ou son sel | 80% |
|---|---|
| anti-histaminique | 20% |

**7.** Moyen de contraste de néoplasmes malins dans leur diagnostic comprenant de la fluorescéine ou son sel en tant qu'agent de contraste, caractérisé en ce que ledit moyen comprend en outre des saccharides et/ou des disaccharides, des vitamines et des anti-histaminiques en pourcentage pondéral suivant:

| fluorescéine ou son sel | 0,1 à 79,6% |
|---|---|
| saccharide et/ou disaccharide | 19,4 à 98,9% |
| vitamines | 0,5 à 80% |
| anti-histaminique | 0,5 à 80% |

**8.** Moyen de contraste selon la revendication 7, caractérisé en ce que ledit moyen contient des saccharides et/ou des disaccharides, des vitamines et des anti-histaminiques en pourcentage pondéral suivant:

| fluorescéine ou son sel | 15% |
|---|---|
| saccharide et/ou disaccharide | 70% |
| vitamines | 10% |
| anti-histaminique | 5% |

**9.** Moyen de contraste selon l'une quelconque des revendications 1 à 8, caractérisé en ce que les sels de fluorescéine sont des sels de métaux alcalins.

**10.** Moyen de contraste selon l'une quelconque des revendications 1 à 8, caractérisé en ce que les sels de fluorescéine sont des sels d'ammonium.

**11.** Moyen de contraste selon la revendication 9, caractérisé en ce que les sels de métaux alcalins de la fluorescéine sont des sels de Li-, Na- et K-.

**12.** Moyen de contraste selon l'une quelconque des revendications 1, 5 à 11, caractérisé en ce que le saccharide est du glucose et/ou du fructose.

**13.** Moyen de contraste selon l'une quelconque des revendications 1, 5 à 11, caractérisé en ce que le disaccharide est du saccharose.

**14.** Moyen de contraste selon l'une quelconque des revendications 3 et 7 à 13, caractérisé en ce que les vitamines sont des vitamines du groupe A et/ou du groupe B et/ou du groupe C et/ou P et/ou PP et/ou E.

**15.** Moyen de contraste selon l'une quelconque des revendications 3 et 7 à 13, caractérisé en ce que les vitamines sont des vitamines du groupe B et/ou du groupe C et/ou P et/ou PP et/ou E.

**16.** Moyen de contraste selon l'une quelconque des revendications 5 et 7 à 15, caractérisé en ce que l'antihistaminique est un 1,5-disulfonate de 3-méthyl-9-benzyl-1,2,4-tétrahydrocarbolin-naphtaline, chlorhydrate de 10-(2-diméthylaminopropyl)-phénothiazine, chlorhydrate de N-diméthylaminoéthyl-N-(para-chlorobenzyl)aminopyridine ou de 1-méthyl-2-[2-($\alpha$-méthylpara-chlorobenzhydryloxy)éthyl]-pyrrolidine, hydrofumarate de 4,9-dihydro-4-(1-méthyl-4-pipéridinylidène)-1-OH-benzo[4,5]cyclohepta[1,2]thiophène-10-OH, chlorhydrate du $\beta$-diméthylaminoéthyléther de benzhydrol ou de chinuklydyl-3-diphényl-carbinol.

**17.** Moyen de contraste selon l'une quelconque des revendications 1 à 16, caractérisé en ce qu'il se trouve sous forme de solution ou sous forme de pulvérisation.

**18.** Moyen de contraste selon la revendication 17, caractérisé en ce que la solution est de l'eau ou une solution aqueuse contenant de 5 à 30% d'alcool éthylique.